# EUROPEAN PATENT APPLICATION

(11) **EP 3 834 853 A1**
(43) Date of publication of application: **16.06.2021**
(21) Application number: 19216118.0
(22) Date of filing: 13.12.2019
(51) Int. Cl.: A61L 27/06, A61L 27/30, A61L 27/32, A61L 27/50, A61L 27/56, A61L 27/10

(54) **COATINGS, COATED IMPLANTS AND MANUFACTURING METHODS THEREFORE**

(71) Applicant: EMPA Eidgenössische Materialprüfungs- und Forschungsanstalt, 8600 Dübendorf (CH); Eidgenössische Technische Hochschule Zürich, 8092 Zürich (CH)
(72) Inventor: Matter, Tino, 8600 Dübendorf (CH); Herrmann, Inge Katrin, 8600 Dübendorf (CH); Pratsinis, Sotiris, 8032 Zürich (CH)
(74) Representative: Grimm, Siegfried

(57) **Abstract**

The present invention relates to novel coatings and its uses, as well as processes of making such coatings. The coatings are characterized by their chemical composition and architecture; they are antimicrobial, tissue regenerative and tissue bonding, making them useful as a coating of an implant. Manufacturing processes for such coating combines flame spray pyrolysis and direct coating of a substrate; the process uses inexpensive starting materials and is scalable.

## Description

The present invention relates to coatings and its uses as well as processes of making such coatings. The coatings are characterized by their chemical composition and architecture; they are antimicrobial, tissue regenerative and tissue bonding, making them useful as a coating of an implant. Manufacturing processes for such coating combine flame spray pyrolysis and direct coating of a substrate; the process uses inexpensive starting materials and is scalable.

**Flame Spray Synthesis** (FSP) is a well-known method for manufacturing nanoparticulate materials.
WO2004/005184 discloses the synthesis of metal oxides via FSP. The thus obtained nanoparticulate material may be used in catalytic applications.
WO02005/087660 discloses the synthesis of metal salts, particularly calcium phosphate, via FSP. The thus obtained nanoparticulate material is biocompatible and osteoconductive and may be used in medical applications. WO2011/020204 discloses the synthesis of radio-opaque bioactive glass materials via FSP. The thus obtained nanoparticulate material may be used in dental fillings. The FSP methods disclosed in these documents are suitable for a wide range of applications, but fail to prepare structured coatings as described herein.

**Implants** for medical applications are well-known. It is generally accepted to improve their performance by partially or fully coating their surface.
WO2017/062573 discloses nanoceria coated implants to reduce osteolysis. The coating is obtained by wet-phase synthesis. Although suitable, the manufacturing of such coatings is difficult to control and thus less suited for commercial manufacturing. The manufacturing disclosed therein fails to prepare structured coatings as described herein.
WO2017/031524 discloses porous crystalline hydroxyapatite films. The films may be obtained by FSP and may be used to manufacture a prosthesis. Although suitable, the films disclosed in that document suffer from limited bioactivity and slow resorption rate.

In consequence, there is a need for providing improved methods to manufacture nanostructured coatings and a need for providing improved implants.

Thus, it is an object of the present invention is to mitigate at least some of these drawbacks of the state of the art. In particular, it is an aim of the present invention to provide improved coatings, particularly coatings that meet with one or more of the following (i) easier manufacturing, (ii) more reliable manufacturing, (iii) more flexible manufacturing. It is a further aim to provide improved implants, particularly implants that increase the success rate of implantations. Such superior properties may be achieved by one or more of the following (i) better cell attachment on the implant, (ii) decreased foreign body reaction and inflammation (iii) higher cytocompatibility and/or (iv) preventing formation of biofilms on the implant.

These objectives are achieved by a coating as defined in claim 1; an implant as defined in claim 6 and its manufacturing as defined in claim 10. Further aspects of the invention are disclosed in the specification and independent claims, preferred embodiments are disclosed in the specification and the dependent claims.

The present invention will be described in more detail below, wherein in a **first aspect,** new coatings are described; in a **second aspect,** implants comprising such coatings are described ; and in a **third aspect,** the manufacturing of such new coatings and implants is described.
According to the present invention, stable and diverse nanoparticle coatings are provided; they are obtainable by using fast, inexpensive and scalable flame-spray pyrolysis.
Improved bone integration of implants possessing the inventive coating is observed. Without being bound to theory, it is believed that the biomineralization of bioglass taking place on the coating's surface supports such beneficial effect.
Further, the inventive implants allow improved cell adhesion on their surface. Without being bound to theory, it is believed that this beneficial effect is due to the high compatibility of the inventive coatings with gingival fibroblasts.
Still further, the inventive implants prevent infections after implantation. Without being bound to theory, it is believed that the incorporation of ceria into the coating supports antimicrobial properties.

Unless otherwise stated, the following **definitions** shall apply in this specification:
It is understood that the various embodiments, preferences and ranges as provided / disclosed in this specification may be combined at will. Further, depending of the specific embodiment, selected definitions, embodiments or ranges may not apply.

As used herein, the term "a", "an", "the" and similar terms used in the context of the present invention (especially in the context of the claims) are to be construed to cover both the singular and plural unless otherwise indicated herein or clearly contradicted by the context. As used herein, the terms "including", "containing" and "comprising" are used herein in their open, non-limiting sense. The term "containing" shall cover "comprising", "essentially consisting of" and "consisting of".

The "diameter" of a particle as described herein is the volume-surface-average diameter of the primary particle. This may be determined by nitrogen adsorption using the BET method (according to: Janssen et al, Journal of Applied Polymer Science 52, 1913, 1994).

The term "Bioactivity" or "bioactive material" is known in the field and relates to the effect of a material upon a living organism. A specific manifestation thereof is "biomnieralisation". Briefly, biomineralisation refers to the ability of a material to induce calcium phosphate deposition on the surface of the object when placed in fluids. These fluids can be but are not limited to simulated body fluid, phosphate buffered or unbuffered physiological sodium chloride solution and the fluid in a living organism.

The term "Biodegradation" or "biodegradability" is known in the field. Briefly, the term is used herein to relate the degradation of materials by humidified atmospheres and/or by liquid at various temperatures and/or cellular action. The characteristic parameter for the degradation is the mass loss of the material and/or the reduction in molecular weight of the applied material.

The term "Primary Particle" is known in the field: Briefly the term relates to particles in the nanometer scale, such particles are often termed "nanoparticles". Suitable are particles between 5 - 200 nm, preferably 5 - 90 nm, particularly preferably 5 - 20 nm. Such particle size results in material having a high surface area (e.g. > 30 m²/g). This allows a homogeneous distribution in a formulation as defined below and improved quality during X-ray investigations. Nanoparticles, in the context of this invention, comprise primary particles and secondary particles. The term secondary particle includes such particles which are formed by interaction of primary particles. These secondary particles can be significantly larger than primary particles. Secondary particles can be formed by primary particles attached to each other by physical forces, such as Van-der-Waals forces, or by individual primary particles grown together which have solid necks between the individual primary particles and can be formed by Oswald ripening or sintering / annealing. The 3-D fractal dimension of the secondary particles can vary over a wide range from just above 1 to nearly 3.

The present invention will be better understood by reference to the **figures.**

### List of references:

(1) Coating
(2) Nanoparticles, (21) first group; (22) second group, (23) third / further group of nanoparticles
(3) Substrate
(4) Implant
(5) FSP apparatus
(6) Flame
(7) Precursor solution, (71) precursor solution for first group; (72) precursor solution for second group, (73) precursor solution for third / further group of nanoparticles. Flame spray pyrolysis allows the production a wide spectrum of metal oxides from inexpensive metal salts.

Fig. 1 shows in sections A, C and D various embodiments for a suitable set-up to manufacture the inventive coatings and devices. In section B, various embodiments of the nanoparticles are shown:
   (i) core - shell type nanoparticle
   (ii) janus - type nanoparticle
   (iii) mixed phases / co-oxidized nanoparticle
   (iv) phase segregated nanoparticle.
Fig. 2 schematically represents an implant (4), in the form of an implant screw for dental applications, having a first and a second coating ((11) and (12)) as well as a non-coated surface (3); schematically shown are regions of hard tissue (ht), soft tissue (st) and for the crown of the implant (cr)
Fig 3: Atomic force micrographs of the uncoated and coated Ti-disk showing deposition of nanoparticles on an implant surface according to examples #0 - #4. a) uncoated, ex#0 . b) ceria coated, #1. c) bioglass coated, #2. d) BG/ceria coated, #3. e) Zn2-SrBG/ceria coated, #4.
Fig. 4: Secondary electron micrographs of nanoparticle coatings on Ti substrates. A trench was introduced by focused ion beam and the coatings were imaged from the side.
Fig. 5: Standardized abrasion measurements on the coated implants. X-axis: no. of cycles; Y-axis m.%.
Fig. 6: Secondary electron micrographs of a bioglass-coated Ti substrate according to #2; scale bar shows 10 micrometer
   (a) directly after preparation
   (b) after 1 day and week immersion in simulated body fluid
   (c) after 1 week immersion in simulated body fluid.
Fig. 7: Cytotoxicity against gingival human fibroblasts of the inventive coatings (3^{rd} to 6^{th} bar); the toxic surfactant Triton X was used as a positive control (1^{st} bar) and non-toxic dPBS buffer as a negative control (2^{nd} bar.
Fig. 8: Antibacterial activity against methicillin-resistant Staphylococcus aureus (MRSA).

In more general terms, in a **first aspect,** the invention relates to a structured coating (1) which is defined by its chemical composition (i.e. the material it is composed of) and by its morphology (also referred to as architecture or geometry). Specifically, the invention provides for a structured coating (1) comprising Bioglass (preferably 5-100 wt%) as a first group of oxidic material and optionally ceria (preferably a 0-95 wt%) as a second group of oxidic material, wherein the coating comprises a plurality of agglomerated nanoparticles (2) of said first and second group of oxidic material (21, 22); and wherein said nanoparticles (2) having a mean particle diameter of 5 - 200 nm as determined by electron microscopy; and wherein the surface roughness of the coating is 100 - 800 nm of Ra as determined by atomic force microscopy and wherein the porosity is at least 60, as determined by electron microscopy. This aspect of the invention shall be explained in further detail below:

### Chemical composition:

The inventive coating comprises one or more chemical entities, whereby bioglass is a mandatory component (first group of oxidic material), ceria is an optional component (second group of oxidic material) and a third component may further be present. As described in further detail below, the chemical composition of the inventive coatings may vary over a broad range. This is considered a significant benefit, as the coating may be adapted (or tailored) to the need of a specific implant.
In one embodiment, the inventive coating comprises, particularly consists of, bioglass. Accordingly, the inventive coating may comprise up to 100 wt% bioglass.
In one further embodiment, the inventive coating comprises, particularly consists of, a combination of both, bioglass and ceria. Accordingly, the inventive coating may comprise less than 100 wt% bioglass and more than 0 wt% ceria. In an advantageous embodiment, the invention relates to a coating comprising 20-90 wt%, such as 30-80 wt% bioglass and 10-80 wt%, such as 30-70, wt% ceria.
In one further embodiment, the inventive coating comprises, particularly consists of, a combination of three chemical entities, bioglass, ceria and a third component. Accordingly, the inventive coating may comprise less than 100 wt% bioglass, more than 0 wt% ceria and more than 0 wt% of said third component. Such third component may comprise oxidic material functioning as a connective layer, such as zirconia. Said third component may amount to up to 90 wt%. In an advantageous embodiment, the invention relates to a coating comprising 20-90 wt%, such as 30-80 wt% bioglass, 5-75 wt%, such as 10-60 wt% ceria and 5-75 wt%, such as 10-60 wt% of said third component.

**Bioglass (1^{st} group of material, BG) :** This material is known in the field, also termed bioactive glass, and discussed in e.g WO2011/020204. It is a bioactive ceramic material that forms a hydroxyapatite layer in physiological media and releases Ca ions. Bioglass is prepared from Ca salts and further components and thus distinguishes from Ca salts. Without being bound to theory, it is believed that released ions locally increase tissue regeneration via different pathways. It is further believed that collagen binds on the surface of bioglass particles that makes them effectively connect different tissues. The term "bioactive" particularly refers to the ability of these particles / compositions to bond bone and / or soft tissue. Suitable bioactive glasses contains a matrix of silicon oxide and additional oxides. Examples of additional oxides include, but are not limited to, sodium oxide, calcium oxide, phosphorous oxide, aluminium oxide, boron oxide, strontium oxide, potassium oxide, magnesium oxide or combinations and mixtures thereof. Typically Bioglass contains (i.e. comprises or consists of) oxides of Si, Ca / Sr, Na and P and optionally further dopants.

In one embodiment, the bioglass contains oxides of Si, Ca Na and P and optionally further dopants, but no oxides of Sr.
In one embodiment, the bioglass contains oxides of Si, Sr Na and P and optionally further dopants, but no oxides of Ca.
In one embodiment, the bioglass contains oxides of Si, Ca, Sr, Na and P and optionally further dopants.
Bioglass typically contains SiO2 in an amount of less than 66wt%, such as 20 - 60 wt%, preferably 30 - 50 wt%.
Bioglass typically contains CaO or SrO (as the case may be) in an amount of 10 - 50 wt%, preferably 20 - 40 wt%. Bioglass typically contains Na2O in an amount of 5 - 50 wt%, preferably 15 - 30 wt%.
Bioglass typically contains P2O5 in an amount of 0 - 20 wt%, preferably 3 - 10 wt%. It is believed that P205 supports nucleation of calcium phosphate, which deposits on the surface; it is not considered essential.
Bioactive glasses distinguish from calciumphosphates in that (i) silica is present, (ii) the amount of sodium is higher (aqueous solutions thereof show a high ph value) and (iii) phosphorous is an optional component.
In an advantageous embodiment of the present invention, the ratio Ca:P (or Sr : P or (Ca+Sr) : P) in the inventive particle is in the range of 10:1 to 1:10.
In an advantageous embodiment of the present invention, the ratio Ca:Na (or Sr:Na or (Ca+Sr) :Na) is in the range of 2:1 to 0.5:1.
Bioglass may further contain dopants ("BG-dopants"), typically in an amount of less than 5 wt%. Suitable dopants include Ag, Cu, Ga, Zn and Co. Such BG-dopants may be present in metallic and / or oxidic form.
Examples of Bioglass include types 45S5, 42S5, S53P4, 55S4, 58S, 70S30C, 45S5F, 40S5B5, all of them being known per se; chemical compositions may be taken from the following table:

| **Bioglass** | **SiO₂** | **Na₂O** | **CaO** | **P₂O₅** | **Other** |
|---|---|---|---|---|---|
| 45S5 | 45 | 24.5 | 24.5 | 6 | - |
| 42S5 | 42.1 | 26.3 | 29 | 2.6 | - |
| S53P4 | 53 | 23 | 20 | 4 | - |
| 55S4 | 52.1 | 21.5 | 23.8 | 2.6 | - |
| 58S | 60 | 0 | 36 | 4 | - |
| 70S30C | 70 | 30 | 0 | 0 | - |
| 45S5F | 45 | 24.5 | 12.25 | 6 | 12.5 CaF₂ |
| 40S5B5 | 40 | 24.5 | 24.5 | 6 | 5 B₂O₃ |

**Ceria (2^{nd} group of material):** The material is known in the field and discussed in e.g. WO2004/005184.
It possesses antioxidative properties and neutralizes reactive oxygen species by mimicking the body's own enzymes. Additionally, ceria possesses antimicrobial properties. Without being bound to theory, it is believed that the above properties support wound healing and mitigate inflammation.
In one embodiment, said ceria is selected from compounds of formula (I)

CeO₂₋ₓ (I)

wherein x is between 0 and 0.67; and wherein up to 10 wt% of Ce may be replaced by a dopant metal ion.
In one embodiment, said ceria is stoichiometric cer(IV)oxide; x=0. It may be crystalline or amorphous, preferably crystalline.
In one embodiment, said ceria is nonstoichiometric cer(IV)oxide; 0.2≥x>0. It may be crystalline (with crystal defects) or amorphous, preferably crystalline.
In one embodiment, said ceria is stoichiometric cer(II,III)oxide [Ce3O4]; x=0.67 It may be crystalline or amorphous, preferably crystalline.
In one embodiment, said ceria (stoichiometric or nonstoichiometric) may contain dopants ("ceria-dopants"). Suitable dopants are known in the field and include Zr, Zn, Cu, Ag and Ga, preferably Zr. Such Ceria-dopants may be present in metallic and / or oxidic form. The amount of Ceria-dopant may vary over a broad range, typically below 10 wt%.
As indicated by formula (I), stoichiometric and non-stochiometric ceria is covered. This also includes surface defects which are observed for nanoparticulate material, particularly when obtained by an FSP process.

**Third Component** (3^{rd} group of material): In a further embodiment, one or more types of other particles, typically also in the nanometre scale as defined herein, may be present in the inventive coatings.
By way of example, zirconia may be identified as a third component (23). In an advantageous embodiment, a zirconia-rich region of the inventive layer functions as a connective component towards a substrate (as defined below) while a bioglass/ceria rich region functions as a connective component towards tissue.

The above named materials, bioglass, ceria, third component, are known per se and may be obtained according to the FSP process described below, second aspect of the invention.

### Morphology / Architecture:

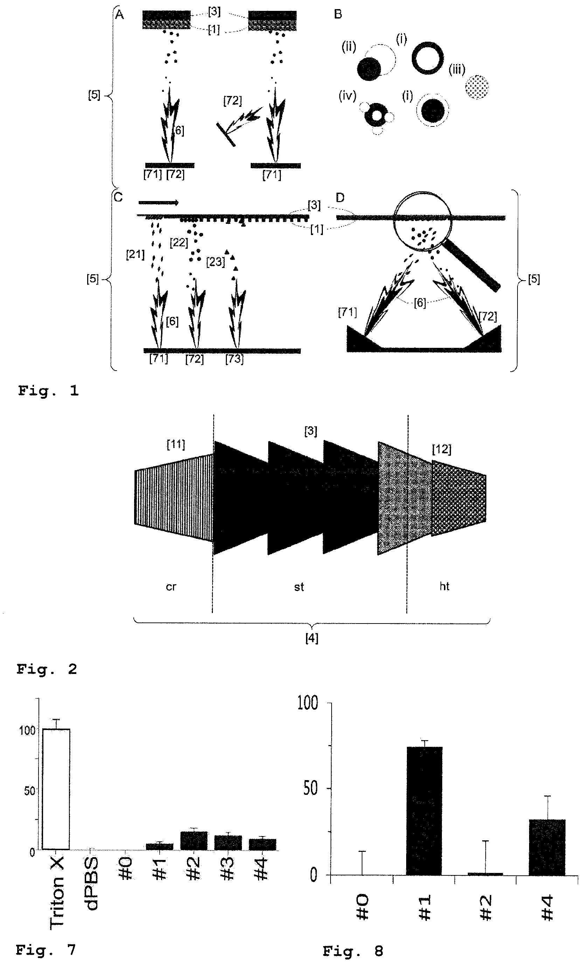

Architecture and structure significantly influence properties of the inventive coatings. The architecture may be specified on various levels: macroscale, nanoscale and single unit.
a. **Macroscale:** On a macroscale, the substrate may be coated differently. In one embodiment, two or more sections (e.g. two sides) of the substrate's surface are differently coated; In one alternative embodiment, the substrate's surface is coated with a coating showing a gradient e.g in thickness and or in chemical composition. In one further embodiment, the substrate's surface is coated with a patterned coating (e.g. showing a pattern coated / non-coated or showing a pattern of its chemical composition). Such variation of the coating on a macroscale results in implants where different tissues experience different surfaces. This finds use for example in tooth implants where one side is anchored in soft tissue (gum) and the other in bone (tooth).
b. **Nanoscale:** The nanostructure of the surface has a specific topography, porosity and roughness. The inventive coating comprises a plurality of nanoparticles that are partly or fully agglomerated. While the particles produced during the inventive process (the flame spray pyrolysis described below as step (c)) are primarily or exclusively "primary particles", they become partly or fully "agglomerated" upon formation of a coating (the coating process described below as step (d)). Primary particle size is as discussed above, preferably around 5-20 nm, but they are at least partly sintered to form agglomerated particles.
   In one embodiment, the surface roughness of the coating is 100 - 800 nm, preferably 150-400 nm as determined by AFM; and / or
   In one embodiment, the particle size is 5-90 nm, such as 5-20 nm as determined Electron Microscopy; and / or
   In one embodiment, the thickness of the coating is 200 - 10000 nm, preferably 1000 - 5000 nm, as determined by Scanning Electron Microscopy; and/or
   In one embodiment, the porosity of the coating is >60%, preferably >70%, much preferably <75%, as determined by electron microscopy.
c. **Single unit:** The single units of the coating (nanoparticles, agglomerated nanoparticles, (2)) have complex architectures themselves and combine properties on the nanoscale. They consist of multiple metal-oxide phases (e.g. bioglass and ceria) in different arrangements (pure, core-shell, janus types). Additionally, they contain dopant ions dispersed in their main materials. This situation is visualized in Fig. 1B. Accordingly, the invention provides for a coating wherein said first group of oxidic material and said second group of oxidic material (i) are present as separate nanoparticular entities or (ii) are present as combined nanoparticular entities (including core-shell particles and janus-type particles).

The inventive coating comprises particles having a narrow primary particle size distribution. The distribution of the particle diameter is evaluated by measuring the particle diameters of at least 200 individual, representative particles from transmission electron micrographs. The particle size distribution is then fitted by a log-normal distribution (see Grass and Stark, Journal of Materials Chemistry 2006 Vol. 16, P 1825 ff). The distribution is characterized as narrow if the geometric standard deviation of the measured and fitted distribution is below 1.9, more preferably below 1.7 and most preferably below 1.5. A narrow size distribution, as described above, improves the quality of the material and simplifies the uses disclosed herein.
Particle size may vary for the 1^{st}, 2^{nd} and 3^{rd} group of material. This will result in a mono-modal or bimodal or three-modal size distribution.

### Medical applications:

As the coatings described herein have beneficial biochemical properties, they are useful in in medical applications, particularly for coating of implants The invention thus also provides for the use of a coating as described herein in the manufacture of an implant for the treatment of hard tissue of a mammal, particular a human; or (ii) the treatment of soft tissue of a mammal, particularly a human; or (iii) the combined treatment of hard and soft tissue of a mammal, particularly a human. In a **Second aspect,** the invention relates to **implants** (4) comprising a substrate (3) having one or more structured coatings (1) as described herein which coated at least partially thereon. As the coatings described herein have beneficial biochemical properties, they are useful in in medical applications, particularly for coating of implants. This aspect of the invention shall be explained in further detail below:

**Implant:** The term is known in the field and particularly relates to a device that is placed inside or on the surface of the body. Many implants are prosthetics, intended to replace missing body parts. Other implants deliver medication, monitor body functions, or provide support to organs and tissues. For teeth for example, the screw that goes into the gum is considered an implant whereas the crown is considered a prosthesis. Inventive implants (4) comprise a substrate (3) and are partly or fully coated with a coating (1) as described herein. It is understood that an implant is a man-made device, thereby distinguishing from a transplant, which is a transplanted biomedical tissue.
In the context of this invention, an implant is preferably a device which is anchored in tissue and thus termed medical implant. The term medical implant thus refers to a device fully surrounded by tissue or partly surrounded by tissue. Examples of medical implants include dental implants (e.g. the above screw), artificial hip joints, cardiatic stimulators.
In some cases implants contain electronics (such as an artificial pacemaker) or pharmaceutically active ingredients.

**Substrate:** The substrate contains a material known in the field, including metals, such as titanium and its alloys, iron alloys, and ceramics, such as zirconia. The substrate may be pre-treated, e.g. etched, polished or pre-coated. A pre-coated substrate is preferred in case the inventive coating consists of first group of oxidic material (21) or consists of first and second group of oxidic material (21, 22). (ie. no third component (23).
A non-coated substrate may be used in case the inventive coating consists of (21) and (23) or (21), (22) and (23).

**Coating:** The coating of an implant is known per se, it includes partial and full coating, as well as mono-layer and multi-layer coating.
In one embodiment, the substrate's surface is completely coated with a coating as described herein ("full coating"). In one embodiment, the substrate's surface is partly coated with a coating as described herein ("partial coating"). Accordingly, in one embodiment, the structured coating (1) is present on less than 50% of the substrate's surface or the structured coating is present on more than 90% (preferably 100%) of the substrate's surface.

In one embodiment, the coating is a mono-layer coating. A mono-layer is a layer having the same chemical composition along a line perpendicular to the substrate's surface.
In one embodiment, the coating is a multi-layer coating. A multi-layer is a coating where the chemical composition varies along a line perpendicular to the substrate's surface.
In one embodiment, any one of the parameters particle size, roughness, thickness, porosity varies over the implant's surface.
Such different types of coating are important to meet the specific needs of the implant in question. The inventive manufacturing method described herein (3^{rd} aspect of the invention) readily allows such various coatings.
Without being bound to theory, it is believed that the inventive coating acts as a connective layer between substrate and tissue, allowing for a time-dependent dissolution / reorganization of the implants top layer(s).
For example, a implant's top layer may dissolve over time allowing direct contact of tissue with a lower
In one embodiment, the implant is free of intermediate layers between substrate (3) and structured coating (1). In one embodiment, the implant is free of additional layers on top of the structured coating (1).
In a further embodiment, there is provided an implant as described herein characterized in that (i) the chemical composition of the structured coating (1) varies over the implants surface (x-axis, y-axis); and / or (ii) the chemical composition of the structured coating (1) varies over the coating thickness of the coating (z-axis, the z-axis being an axis perpendicular to the implant surface). These spatial variations on the coating may be obtained according to the methods described below.

**medical use:** In a further embodiment, the invention relates to an implant described herein adapted to (i) the treatment of hard tissue of a mammal, particular a human being; or (ii) the treatment of soft tissue of a mammal, particularly a human being; or (iii) the combined treatment of hard and soft tissue of a mammal, particularly a human being. Accordingly, the invention also provides for the use of an implant as described herein in the above treatments (i), (ii) and (iii). Further, the invention provides for a method of using an implant as described herein in the above treatments (i); (ii), and (iii) in a subject in need thereof.

In a further embodiment, the invention relates to an implant described herein adapted to dental applications in a human. Accordingly, the invention also provides for the use of an implant as described herein in dental applications. Further, the invention provides for a method of using an implant as described herein in dental applications in a subject in need thereof.

Without being bound to theory, it is believed that the inventive coatings, due to their unique chemical composition and morphology, exhibit different properties that increase the success rate of implantations. Fist, better cell attachment on the implant allows for better resorption. Second, decreased foreign body reaction and inflammation results in better acceptance of the body. Third, a higher cytocompatibility is observed. Forth, formation of biofilms on the implant is prevented. This prevention is governed by contact of bacteria with the nanoparticulate coating.

In a **third aspect,** the invention relates to a process for manufacturing a coating and / or an implant as described herein. The inventive method combines FSP synthesis of bioglass and ceria and its direct coating on a substrate. Broadly speaking, the inventive manufacturing process is a one-step process in which one or more solutions (71, 72) are sprayed and ignited and the thus formed nanoparticulate material (21, 22) is directly deposited on a substrate (3) to form a coating (1). Specifically, the invention provides for the manufacturing of a coated implant (4) as described herein, the method comprising the steps of:
(a) providing one or more substrate(s) (3);
(b) providing one or more precursor solutions for bioglass (71) and one or more precursor solutions for ceria (72) and optionally one or more precursor solutions for the third component;
(c) subjecting the solutions (71) and (72) and optionally (73) to an flame spray pyrolysis process to form nanoparticulate material (21, 22(;
(d) directly ("in situ") coating said substrate (3) with the thus obtained nanoparticulate material; and optionally
(e) post - treatment of the thus obtained coated implant. The manufacturing process is scalable, reliable and uses inexpensive starting materials; making its suitable for commercial manufacturing. This aspect of the invention shall be explained in further detail below:
   **Step (a), substrate:** The substrate to be coated may be provided in a fixed position or in a movable position. It may be beneficial to adjust distance between flame and substrate. Such adjustment may affect agglomeration of the nanoparticles and bonding to the surface.
      Further, it may be beneficial to allow rotational movement of the substrate and / or longitudinal movement of the substrate. This may allow selective coating (such as partial or full coating, one or more layers) of the substrate.
      The substrate may be provided with features for temperature control thereof, particularly for cooling. Advantageously, step (a) comprises providing as substrate (3) and means for contact cooling. Such cooling improves step (d) in that sintering of the nanoparticulate material on the substrate is controlled.
      As discussed above, the substrate may native or may be pre-treated. Pre-treatment may increase surface roughness, e.g. by etching or sandblasting.
   **Step (b), precursor solutions:** The starting material for step (c) is provided in the form of one or more solutions comprising one or more metal / phosphor precursors and one or more solvents.
      Preferably, precursor solutions are combustible. This facilitates step (c). Suitable solvents (including combination of solvents) have a net heat of combustion of > 15 kJ/g, preferably > 20 kJ/g, more preferably > 23 kJ/g. This may be achieved by providing an organic solvent or a mixture of organic solvents with a mean number of carbon atoms of > 2 C atoms, usually at least 2.2 C atoms, preferably at least 3 C atoms, more preferably about 4 to 10 C atoms. Suitable solvents may be selected from the group consisting of aliphatic hydrocarbons, aromatic hydrocarbons, alcohols, ethers, esters, and carboxylic acids.
      Preferably, the precursor solution has a viscosity of < 100 mPas, preferably, < 40 mPas, much preferably < 20 mPas. This low viscosity, such as 0.3 - 100 mPas, facilitates step (c).
      Suitable precursor compounds for the use in the method of the present invention are compounds that are soluble in a solvent as outlined above. Although such precursors can be any sufficiently stable salts, organic groups comprising salts are preferred, in particular purely organometallic compounds or organometalloid compounds such as a salt of at least one (optionally substituted) carboxylic acid, such as acetic acid, formic acid, but also dicarboxylic acid, oligocarboxylic acid and/or polycarboxylic acid and/or other common organometallic or organometalloid ligands such as acetylacetonate, tetramethylacetoacetonate, ethylene diamine and others, optionally as hydrate. The salt may also be produced within the solvent mixture in situ, meaning that a suitable salt precursor (namely a metal comprising compound, e.g. an oxide, a carbonate or a pure metal, that reacts with at least one of the components of the solvent to form a solution) is brought into the solvent mixture where it then forms the salt or derivative of the solvent (e. g. a carboxylic acid salt of a carboxylic acid from the solvent).
      Accordingly, said one or more precursor solutions contain
      - one or more soluble silica precursors, such as hexamethyldisiloxane, tetraethoxysilane or any other organo-silicon compounds;
      - one or more soluble sodium precursors, such as sodium 2-ethylhexanoate or any kind of soluble sodium source such as sodium carboxylate;
      - optionally one or more soluble calcium precursors, such as calcium 2-ethylhexanoate or any kind of soluble calcium source such as calcium carboxylate;
      - optionally one or more soluble strontium precursors, such as Sr 2-ethylhexanoate or any kind of soluble Sr source such as Sr carboxylate;
      - optionally one or more soluble phosphorous precursors, such as tributyl phosphate or any other soluble phospho-rous source such as organophosphorous compounds;
      - optionally one or more soluble precursors for bioglass-dopants, such as BG-dopants described herein;
      - one or more soluble ceria precursors, such as actetate, acetylacetonate, 2-ethylhexanoate or any other organo-cer compound;
      - optionally one or more soluble precursors for ceria-dopants, such as 2-Ethylhexanoates or acetates of zinc, strontium, copper, silver, gallium
      - optionally one or more soluble zirconia precursors, such as actetate, acetylacetonate, 2-ethylhexanoate or any other organo-zirconium compound.
   **Step (c), Flame Spray Pyrolysis: FSP** is a process known per se and described in the above named documents, WO2004/005184 and WO2011/020204. FSP processes are used in industry; they are reliable and scalable. Briefly, nanoparticulate metal oxides are obtained by this method in that the precursor solution of step (b) is formed into droplets and flame oxidized, optionally in the presence of an oxidizing gas. By using multiple spray or flames, as explained below and shown in the figures, complex geometries and architectures of both the nanoparticles and the coatings are obtained.
      Contrary to traditional FSP processes, the thus obtained nanoparticulate material are directly ("in situ") brought into contact with a substrate, step (d) below, thereby forming a coating on said substrate.
      Flame: In the inventive method, the flame has a temperature of at least 1000°C, usually at least 1500°C, preferably at least about 2000°C. A preferred range of the flame temperature for many applications is 1600 to 2600°C. Droplets: Droplets are typically formed by way of one or more spray nozzles. The average diameter of the droplets may vary depend on the liquid dispersion setup and the properties of the liquid itself. Usually, the average droplet diameter ranges from 0.1 µm to 100 µm, preferably from 1 µm to 20 µm.
      Oxidizing gas: Suitable gases are known in the field, premixed methane / oxygen or separate feeds of air and methane may be used.
      The distance flame nozzle - substrate may be adjusted according to the specific requirements of the process. Typically, 2-100 cm, such as 5-50 cm, are a suitable range.
   **Step (d) Coating:** The as obtained nanoparticulate material (bioglass, ceria and optional third component) are directly (in situ) coated on a substrate. Such in situ deposition, in conjunction with the stoichemetric formation of said nanoparticulate material, allows full control over particle composition at different times and thus thicknesses and architecture of the inventive coating. may be subject to a simultaneous coating (c.f. fig....) or a subsequent coating (stepwise coating, fig....). This allows realizing complex geometries and architectures of the inventive coatings.
      Advantageously, the substrate's temperature is controlled, depending on the substrate's material and the coating. Typically, the temperature is in the range of 100 °C to 1500 °C. For metallic substrates, 100 - 800°C is preferred, for ceramic substrates 500 - 1500°C is preferred.
   **Step (e), post-treatment:** Post-treatment steps are optional and known in the field. In one embodiment, the implant obtained in step (d) is subjected to annealing. Annealing may influence agglomeration of nanoparticles. In one embodiment, the implant obtained in step (d) is subjected to ultra-sonication Ultra-sonication may influence porosity of the coating.

**Product-by-Process:** In a further embodiment, the invention relates to coatings and implants as disclosed herein which are obtainable by, or obtained by, a method as described herein.

To further illustrate the invention, the following **examples** are provided. These examples are provided with no intend to limit the scope of the invention.

### I. Synthesis of Nanoparticles and Coatings

The experimental set up for 1^{st}, 2^{nd} and 3^{rd} series of experiments is schematically shown in fig. 1A; the nanoparticles obtained are predominantly of structure (iii) and (iv) as shown in fig. 1B.

### I.1: 1^{st} series of experiments:

All particles were produced by liquid-feed flame spray pyrolysis. The precursors were dissolved in the respective solvents (2-Ethylhexanoic acid: 2-EHA, Tetrahydrofuran: THF) such that the total metal ion concentration of the solution was 0.4 M. 5 mL/min precursor solution was pumped to a water-cooled spray nozzle and dispersed by 5 L/min 02. The pressure drop at the nozzle tip was approximately 1.5 bar. The precursor aerosol was ignited by premixed CH4/O2 (1.5:3.2 L/min) flamelets.

Particles were either collected on a glass fiber filter mounted approx. 70 cm above the nozzle to obtain bulk material of the particles or on a titanium disk mounted approx. 8 cm above the nozzle to obtain the inventive coating.

The following particles and coatings were prepared:
#1. Ceria: 100 wt % cerium(III) 2-ethylhexanoate (49%, in 2-EHA) in THF.
#2. Bioglass: 32.65 wt % calcium acetylacetonate, 41.21 wt % sodium 2-ethylhexanoate, 7.06 wt % tributyl phosphate, and 19.07 wt % HMDSO in THF.
#3. Bioglass and ceria hybrid particles (BG/ceria): Mixture of equal volumes of Bioglass and ceria precursor as described above.
4. Bioglass and ceria hybrid, matrix substituted with strontium oxide (SrO) and doped with zinc (Zn2-SrBG/ceria): 5.01 wt % calcium acetylacetonate, 13.02 wt % sodium 2-ethylhexanoate, 2.04 wt % tributyl phosphate, 6.10 wt % HMDSO in THF mixed with 3.85 wt% Strontium acetylacetonate hydrate, 68.34 wt% cerium(III) 2-ethylhexanoate (49%, in 2-EHA), 1.64 wt% Zinc acetylacetonate in 2-EHA.

### I.2_{:} 2^{nd} series of experiments:

In a further series of experiments, the Ti substrate was replaced by Yttrium-stabilized zirconia. The results obtained with such substrate are comparable to the here described results with Ti.

### I.3: 3^{rd} series of experiments:

In a still further series of experiments, the Ti substrate was cooled upon in situ coating. The results obtained with such method are comparable to the here described results with non-cooled substrate. This modified method prevented substrate damage.

### II. Analytical data

The analytical data provided herein refer to this 1^{st} series of experiments.

### II.1. Atomic Force Microscopy

Surface roughness was measured by Atomic Force Microscopy (AFM, Nanosurf). The Dyn190Al cantilever was operated at a frequency of 190 kHz, with a force constant of 48 N/m. Image analysis was conducted using Gwyddion.

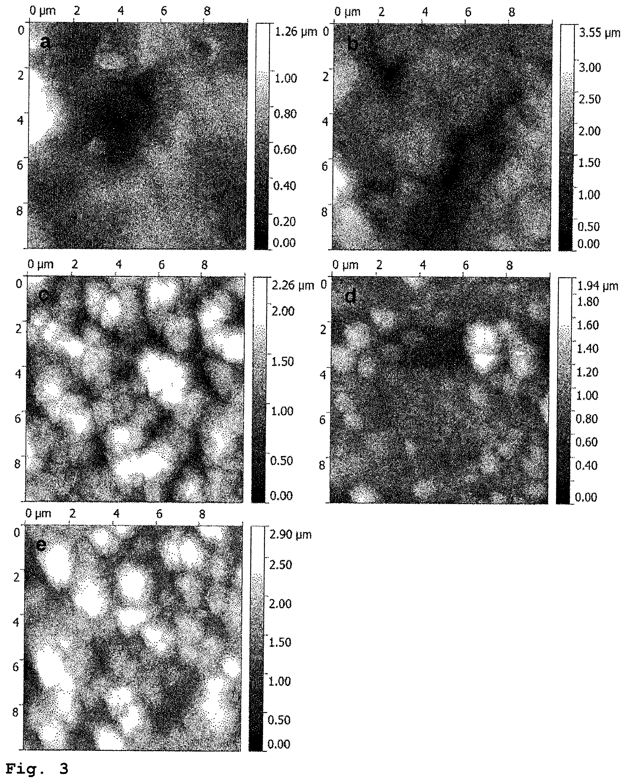

The results are shown in fig. 3. The micrographs show a well distributed layer of agglomerated nanoparticles on the surface of the Ti substrate.
Coating roughness (calculated from the AFM data shown in Fig. 3) and contact angle of the thus obtained coatings are provided in the table below.

| ***Coatings*** | ***Contact angle (°)*** | **Sa *(nm)*** | ***Sq (nm)*** |
|---|---|---|---|
| *#0 no coating* | 68 ± 2 | 98 ± 57 | 145 ± 91 |
| *#1 Ceria* | 8 ± 0 | 660 ± 389 | 602 ± 288 |
| *#2 BG* | 0 | 208 ± 52 | 277 ± 57 |
| *#3 BG*/*ceria* | 0 | 547 ± 170 | 426 ± 129 |
| *#4 Zn2-SrBG*/*ceria* | 0 | 356 ± 59 | 437 ± 64 |

The results show that inclusion of ceria leads to higher roughness. Roughness is an important feature for successful cell adhesion in the body and thus to implant integration.

The results further show that the contact angle was strongly reduced by the coating, meaning the coated implant is more hydrophilic than uncoated. Hydrophilicity is essential for cellular attachment and implant integration.

### II.2 Focused Ion Beam-Scanning Electron Microscopy

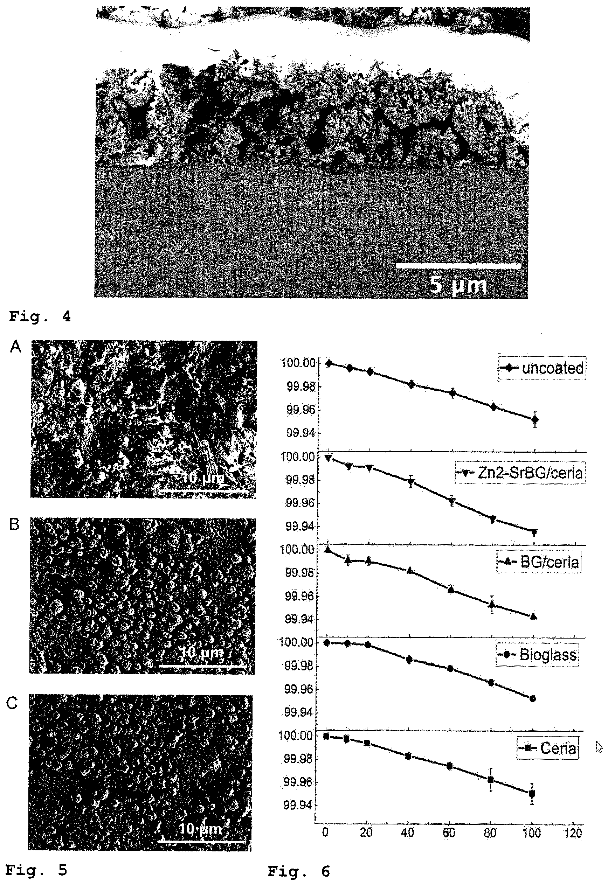

The scanning electron micrographs were acquired using FEI Helios 660 G3 UC FIB/SEM (10 kV, 0.4 nA) equipped with an in-column detector (ICD). Trenches were cut by a focused gallium ion beam. The subsequent images were acquired using an ICD using 5 kV acceleration voltage and 0.1 nA electron beam current.
The results of example #3 are shown in fig. 4. The cross-section shows a stable, yet porous coating on top of the Ti substrate with an average thickness of around 4 µm. Coating thickness is controlled by deposition time and precursor metal ion concentration.

### II.3 Abrasion

Taber Abrader (Model 5135, Taber, North Tonawanda, NY) was used to test abrasion. The applied weight was 1 kg at 60 rpm sample rotation. The abrasive material, a S42 sandpaper strip was wrapped around CS-0 rubber wheel (Taber, North Tonawanda, NY).
The results in fig. 5 show standardized abrasion measurements on the coated implants. The inventive coatings #1 - #4 show similar stabilities as the untreated Ti substrate #0.

### II.4 Biomineralization

Disks were immersed for different times into simulated body fluid according to Kokubo (c.f. Chapter 7 of Handbook of biomineralization, Wiley, 2007, doi.org/10.1002/9783527619443.ch51) .
Then, scanning Electron Microscopy (SEM, Hitachi S-4800) coupled to Energy Dispersive X-ray (EDX) was performed. Cells morphology was investigated by SEM. Briefly, cells were fixed using Karnovsky solution (4% paraformaldehyde, 2.5% paraformaldehyde) for 1 h. After dehydration in an ascending ethanol series and drying with hexamethyldisilazane, a gold film was deposited on the samples by sputtering at 2kV acceleration voltage and 10 µA current.
The results of example #2 are shown in fig. 6. There are evident changes in micro-morphology between the three time points (4 hours, 1 day, 1 week). The bioglass mineralizes on top of the implant, an effect that supports integration into bone.
Similar results are observed for examples #3 and #4.

### 11.5 Cytotoxicity

**Cell culture:** HUV-EC-C, a human umbilical endothelial cell line, was cultured using PromoCell Endothelial Cell Growth Medium 2 supplemented with 2% of the PromoCell Endothelial Supplement Mix. The Normal Human Dermal Fibroblasts (NHDF) cell line and the primary Human Gingival Fibroblasts (HGF), were both cultured using Dulbecco's Modified Eagle's Medium, high glucose, (Invitrogen) supplemented with 10% fetal calf serum (Invitrogen) and 1% penicillin, streptomycin and neomycin (Invitrogen). Cells were cultured at 37°C and 5% CO2.
**Cytotoxicity:** Cytotoxicity was assessed by measuring lactate dehydrogenase (LDH) release using CytoTox 96® NonRadioactive Cytotoxicity Assay (Promega). After cell seeding, 50 µL of the medium were mixed with 50 µL of the LDH detection reagent in a 96-well plate. After 30 minutes, the absorbance of the plate was read in a Mithras plate reader at 490 nm.
The results are shown in fig. 7. The toxic surfactant Triton X was used as a positive control and non-toxic dPBS buffer as a negative control. While all coatings show low toxicity, the addition of ceria and strontium to the coating significantly improve compatibility. Compatibility with gingival human fibroblasts indicates good prospects for implant integration into the gums (gingiva). Additionally, the improved adhesion of fibroblasts has been seen in confocal microscopy.

### II.6 Antimicrobial activity

MRSA DMSZ 1107 was grown overnight in nutrient broth medium tryptic soy broth (TSB100) at 37°**C**, 160 rpm. Bacteria were then transferred onto a TSB agar plate to form colonies over-night at 37°**C**, 5% CO2. For each experiment, one colony was inoculated in TSB100 and grown overnight. Then, bacteria were diluted to an optical density at 600 nm (OD600) of 0.1 and grown for 1-1.5 h, to reassure that the bacteria were in log phase. The bacterial culture was subsequently diluted to OD600 = 0.01 (around 5e6 CFU/ml) and 1 ml was added on top of each implant in a 24-well plate. TSB100 containing no bacteria was used as a control experiment on the implants. After incubation for 2.5 h at 37 °C and 160 rpm, 100 µL of each supernatant was collected and diluted 104 times. 100 µL of the diluted suspensions were plated on a count agar dish and incubated at 37 °C until colonies were countable. Colony forming units (CFU) were quantified using the SCAN® 300 with SCAN® Software. The results are shown in fig. 8: Antibacterial activity against methicillin-resistant Staphylococcus aureus (MRSA) was measured. Whereas the uncoated and the bioglass-coated disks show no antibacterial activity, the incorporation of ceria into the coating significantly reduces bacterial growth. Infections are common after implantation and can lead to drastic consequences. The prevention of biofilm formation is pivotal to implantation success and has been achieved here by incorporating ceria into the coating.

### II.7 Confocal Laser Scanning Microscopy

Cells were fixed either with a 4% parafolmadehyde solution or a fixation solution containing 4% parafolmadehyde, 65 mM PIPES, 25 mM Hepes, 10 mM EGTA, 3 mM MgCl2 for 20 minutes. After fixation, cells were permeabilized using a 0.1% Triton X solution for 10 minutes. Cells were stained for actin and nuclei, using Alexa488-phalloidin (1:250, Thermo Fischer) and DAPI (1:1000, Thermo Fischer).
As a result, an improved adhesion and spreading of fibroblasts was observed on the coated implants.

## Claims

1. A structured coating (1) comprising at least 5 wt% Bioglass as a first group of oxidic material and at least 5 wt% ceria as a second group of oxidic material,
wherein the coating comprises a plurality of agglomerated nanoparticles (2) of said first and second group of oxidic material (21, 22); and wherein said nanoparticles (2) have a mean particle diameter of 5 - 200 nm, as determined by electron microscopy; and.
wherein the surface roughness of the coating is 100 - 800 nm of Ra, as determined by atomic force microscopy; and
wherein the porosity is at least 60%, as determined by electron microscopy.

2. The coating according to claim 1, wherein said Bioglass comprises:
▪ Silica, preferably 20-60 wt%;
▪ calcium oxide, preferably 10-50 wt%, but no strontium oxide;
▪ sodium oxide, preferably 5-50 wt%;
▪ optionally phosphorus oxide, preferably 0-20 wt%;
OR
▪ Silica, preferably 20-60wt%;
▪ a combination of calcium oxide and strontium oxide, preferably 10-50 wt%;
▪ sodium oxide, preferably 5-50 wt%;
▪ optionally phosphor oxide, preferably 0-20 wt%;
OR
▪ Silica, preferably 20-60wt%;
▪ strontium oxide, preferably 10-50 wt%, but no calcium oxide;
▪ sodium oxide, preferably 5-50 wt%;
▪ optionally phosphor oxide, preferably 0-20 wt%;
AND
in each case optionally dopants selected from the group consisting of Ag, Cu, Ga, Co and Zn.

3. The coating according to any of claims 1 - 2, wherein said ceria is selected from compounds of formula (I)
CeO₂₋ₓ (I)
wherein x is between 0 and 0.67; and
wherein up to 10 wt% of Ce may be replaced by a dopant, preferably selected from the group consisting of Zr, Zn, Cu, Ag and Ga.

4. The coating according to any of claims 1 - 3, wherein
▪ said nanoparticles (2) have a mean particle diameter of 5 - 90 nm ; and / or
▪ said surface roughness is 150-400 nm; and / or
▪ said porosity is at least 70%; and/or
▪ the thickness of the coating is 200 - 10000 nm, as determined by electron microscopy.

5. The coating according to any of claims 1 - 4, wherein said first group of oxidic material and said second group of oxidic material
▪ are present as separate nanoparticular entities
▪ are present as combined nanoparticular entities (including core-shell particles and janus-type particles)
in each case as determined by electron microscopy.

6. An implant (4) comprising a substrate (3) having one or more structured coatings (1) according to any of claims 1 to 5, coated at least partially thereon.

7. The implant of claim 6 wherein the substrate (3) contains a material selected from the group of metals and ceramics.

8. The implant according to any of claims 6 - 7, **characterized in that**
▪ it is free of intermediate layers between substrate (3) and structured coating (1); and / or
▪ it is free of additional layers on top of the structured coating (1); and / or
▪ the structured coating (1) is present on less than 50% of the substrate's surface or the structured coating is present on more than 90% of the substrate's surface; and / or;
▪ any one of the parameters particle size, roughness, thickness, porosity varies over the implant's surface.

9. The implant according to any of claims 6 - 8 **characterized in that**
▪ the chemical composition of the structured coating (1) varies over the implants surface (x-axis, y-axis); and / or
▪ the chemical composition of the structured coating (1) varies over the coating thickness of the coating (z-axis).

10. A method for manufacturing an implant (4) according to any of claims 6 - 9, comprising the steps of:
(a) providing one or more substrate(s) (3); and
(b) providing one or more precursor solutions for bioglass (71) and one or more precursor solutions for ceria (72) and optionally one or more precursor solutions for a third component (73); and
(c) subjecting the solutions (71) and (72) and optionally (73) to a flame spray pyrolysis ("FSP") process to form nanoparticulate material; and
(d) directly ("in situ") coating said substrate(s) (3) with the thus obtained nanoparticulate material; and optionally
(e) post - treatment of the thus obtained coated implant.

11. The method of claim 10, wherein in step (a)
▪ said substrate is movably mounted; and / or
▪ a multitude of individual substrates is provided; and / or
▪ said substrate is mounted 2-100 cm above the nozzle used in said FSP process; and / or
▪ the angle between the flame used in said FSP process and the substrate surface is 40° to 140°;
and / or wherein in step (b) the viscosity of the precursor solutions lies between 0.3 and 100 mPas;
and / or wherein in step (c) the feed ratio of liquid precursor to oxidizing gas into the flame of said FSP process is between 100 : 1 and 5000 : 1;
and / or wherein in step (d) the temperature of said substrate (3) is controlled, preferably in a temperature range of 100 to 1500 °C;
and / or wherein step (e) comprises annealing and / or ultra-sonication.

12. The method of claim 10 or 11; wherein in step (a) a titanium- or titanium alloy- substrate is provided; and in step (b) a combined precursor solution comprising (71), (72) and optionally (73) is provided.

13. An implant according to any of claims 6-9 adapted to
▪ the treatment of hard tissue of a mammal, particular a human being; or
▪ the treatment of soft tissue of a mammal, particularly a human being; or
▪ the combined treatment of hard and soft tissue of a mammal, particularly a human being.

14. The implant according to claim 14 adapted to dental applications in a human being.
